# FASCICULE DE BREVET EUROPEEN

(11) **EP 3 452 365 B1**
(45) Date de publication et mention de la délivrance du brevet: **23.09.2020**
(21) Numéro de dépôt: 17720752.9
(22) Date de dépôt: 25.04.2017
(51) Int. Cl.: B64D 1/16, A01K 67/033

(54) **DISPOSITIF ET PROCÉDÉ DE LARGAGE DE PRODUITS FRAGILES**
VORRICHTUNG UND VERFAHREN ZUM ABWERFEN VON ZERBRECHLICHEN PRODUKTEN
DEVICE AND METHOD FOR DROPPING FRAGILE PRODUCTS

(30) Priorité: 03.05.2016 FR 1653994
(43) Date de publication de la demande: 13.03.2019
(73) Titulaire: Centre de Coopération Internationale en Recherche Agronomique pour le Développement (CIRAD), 75016 Paris (FR)
(72) Inventeur: BOUYER, Jérémy, 34700 Soubès (FR); SECK, Momar Talla, Dakar BP 2057 (SN); GIMONNEAU, Geoffrey, Bobo-Dioulasso 01 01 BP 454 (BF)
(74) Mandataire: Gevers & Orès
(86) Numéro de dépôt international: PCT/EP2017/059832
(87) Numéro de publication internationale: WO 2017/190991

(56) Documents cités:
- CN-A- 105 197 244
- US-A- 3 118 575
- US-A- 4 260 108
- US-A- 4 537 333
- US-A- 5 148 989
- US-A- 5 794 847
- US-A1- 2015 041 596

## Description

L'invention concerne un dispositif visant au largage dans l'atmosphère de produits fragiles tels que des insectes sans les endommager et en quantité contrôlée. L'invention vise également un procédé de largage mettant en œuvre le dispositif et un moyen de transport équipé d'un tel dispositif.

Le largage d'insectes stérilisés par irradiation ou génétiquement modifiés dans un environnement naturel est devenu un moyen de lutte majeur contre certaines maladies : le paludisme et les arboviroses (Chikungunya, Dengue, Zika) avec les moustiques, les trypanosomoses africaines (maladie du sommeil chez l'homme et Nagana chez les animaux) avec la mouche tsé-tsé.

Les insectes largués sont stérilisés par irradiation ou génétiquement modifiés afin que leur descendance soit non viable. Ils entrent en concurrence avec les insectes naturels et contribuent donc à leur éradication. De la sorte, on évite la diffusion à large échelle de pesticides. Dans d'autres applications, ce sont des parasitoïdes qui sont largués, par exemple des Trichogrammes. Ces parasitoïdes se nourrissent des larves d'insectes nuisibles.

Le largage doit s'effectuer à des quantités précisément déterminées car le dispositif est prévu pour larguer des produits dont on attend un effet sur l'environnement et l'opération de largage doit permettre d'obtenir un effet mesurable sur une zone définie à l'avance.

On connaît plusieurs dispositifs de largage de produits fragiles dans le milieu naturel à des quantités précisément dosées. L'un des moyens consiste à utiliser une vis sans fin. Ce système présente l'inconvénient d'endommager une grande proportion des produits fragiles. On connaît également des dispositifs utilisant une table vibrante. Ces dispositifs sont perturbés par les vibrations des moyens de transport (aéronefs légers) sur lesquels ils sont embarqués et perdent de leur précision. On connaît enfin des dispositifs utilisant un convoyeur à bande qui ne permet pas de réaliser des lâchers de faibles quantités d'insectes et qui blesse les insectes (voir par exemple MXNL05000060). D'autre dispositifs pour le largage de produits fragiles sont décrits dans les documents CN105 197 244 A, US 4260108 A et US 2015/041596 A1.

A cet effet l'invention propose un dispositif pour le largage de produits fragile comprenant :
- un moyen de stockage desdits produits, comportant un moyen d'évacuation desdits produits,
- un moyen de distribution des produits fragiles comportant au moins une cavité ménagée à la surface d'un support, le support étant apte à prendre au moins deux positions, une première position dans laquelle l'au moins une cavité se trouve en face de et à distance dudit moyen d'évacuation et peut se remplir en produits fragiles, et une seconde position dans laquelle ladite cavité est écartée dudit moyen d'évacuation et peut se vider,
ledit dispositif comportant en outre un moyen de mise en communication souple du moyen d'évacuation et de ladite au moins une cavité pour le remplissage en produits fragiles de ladite cavité, ledit moyen de mise en communication définissant un volume reliant le moyen d'évacuation et le support.

Le dispositif comprend principalement un moyen de stockage des produits fragiles à larguer, tel qu'une trémie, et un moyen de distribution des produits fragiles. La trémie comporte classiquement un moyen d'évacuation tel qu'un rétrécissement et un orifice en partie basse en utilisation.

Le moyen de distribution comporte un support muni de cavités à la surface, c'est-à-dire de cavités ouvertes susceptibles d'être remplies puis vidées. Le support peut être animé d'un mouvement, de rotation ou de va et vient comme il sera vu plus loin, de façon, dans une première position, à présenter une cavité en face de l'orifice du moyen d'évacuation de la trémie, et dans une seconde position, à écarter cette cavité de la trémie. Dans la première position la cavité se remplit par gravité de produits fragiles, dans la seconde position elle se vide.

Cette disposition permet en soi d'assurer le contrôle du largage des produits fragiles, car le volume des cavités et la fréquence de leur présentation devant l'orifice de la trémie peuvent être ajustés par l'utilisateur.

La difficulté consiste d'une part à éviter la dispersion des produits fragiles dans la première position du support, lorsque les produits passent de la trémie dans la cavité, et d'autre part à ne pas endommager les produits fragiles lors du passage de la première à la seconde position du support.

A cet effet :
- d'une part le support se trouve à distance du moyen d'évacuation ; on évite ainsi que les produits fragiles soient endommagés par effet de cisaillement entre le moyen d'évacuation et les bords de la cavité,
- d'autre part le dispositif comporte des moyens de mise en communication souples des produits fragiles qui s'étendent entre le moyen d'évacuation et le support ; ces moyens de mise en communication canalisent les produits fragiles de l'orifice de la trémie vers une cavité se trouvant en face de cet orifice.

Selon des caractéristiques de l'invention qui peuvent être prises seules ou en combinaison :
- le moyen de mise en communication peut s'étendent à partir de la périphérie du moyen d'évacuation,
- le moyen de mise en communication peut comporter des composants souples prévus pour assurer le guidage des produits lorsqu'ils tombent dans une cavité se trouvant en face du moyen d'évacuation ; dans un autre mode de réalisation, le moyen de mise en communication est simplement un tube souple,
- l'extrémité libre des composants souples est en contact avec la surface du support, notamment avec la périphérie d'une cavité lorsque cette cavité est en face du moyen d'évacuation ; les moyens de mise en communication sont donc prévus pour balayer la surface du support au cours du mouvement de ce dernier, et ainsi réduire l'effet de cisaillement subi par les produits fragiles,

- les composants souples sont des fils, des filaments, des poils ou des lamelles,
- le support peut être une sphère montée rotative ou un cylindre monté rotatif autour d'un axe,
- l'au moins une cavité peut être de forme hémisphérique,
- le dispositif peut comporter un moyen d'éjection apte à recueillir les produits qui sont sortis d'une cavité et à les guider vers l'extérieur du dispositif,
- le dispositif peut comporter une enceinte formant une forme une barrière thermique, ladite enceinte renfermant le moyen de stockage,
- le dispositif peut comporter en outre un moyen de commande prévu notamment pour ajuster la vitesse de déplacement du support.

L'invention porte également sur un véhicule tel qu'un aéronef comportant un dispositif selon l'invention.

L'invention porte également sur un procédé pour larguer des insectes à l'aide d'un dispositif selon l'invention, comportant les étapes consistant à :
- Remplir le moyen de stockage d'insectes,
- Activer le support de façon à larguer des insectes.

Le procédé peut comporter une étape préalable consistant à refroidir le moyen de stockage de façon à mettre ou maintenir les insectes en léthargie.

Il peut également comporter une étape préalable consistant à embarquer le dispositif dans un véhicule tel qu'un aéronef de façon à effectuer le largage d'insectes en vol.

Les insectes sont avantageusement stérilisés par irradiation ou génétiquement modifiés afin que leur descendance soit non viable.

Des modes de réalisation et des variantes seront décrits ci-après, à titre d'exemples non limitatifs, avec référence aux dessins annexés dans lesquels :
La figure 1 représente schématiquement un dispositif en élévation,
La figure 2 représente une vue agrandie de la zone de capture des produits,
Les figures 3A et 3B représentent schématiquement en coupe un autre mode de réalisation de l'invention.

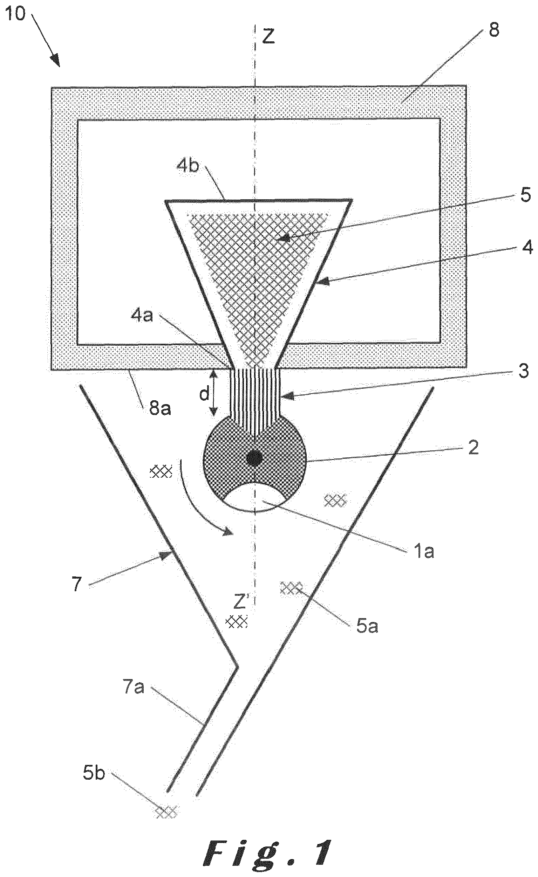

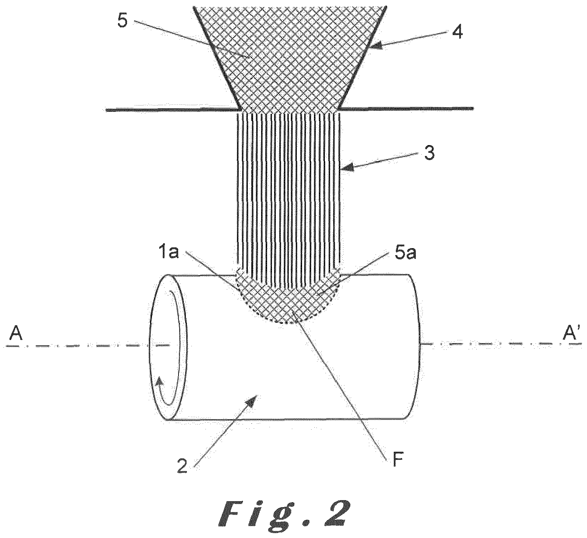

Les figures 1 et 2 représentent un dispositif 10 de largage de produits fragiles 5, par exemple des insectes. Le dispositif 10 est représenté dans sa position d'utilisation, les notions de haut et bas, inférieur et supérieur feront ultérieurement référence à cette position.

Les produits 5 sont stockés dans une trémie 4 d'axe Z-Z' vertical en utilisation présentant, tel que représenté, la forme d'un entonnoir et comportant un moyen d'évacuation 4a sous la forme d'un orifice en partie inférieure, et un couvercle 4b en partie supérieure. Cette trémie 4 est renfermée dans une enceinte 8, avantageusement une enceinte prévue pour maintenir une température de consigne, comme il sera détaillé plus bas. L'orifice 4a débouche sur la face inférieure 8a de l'enceinte, ce qui permet d'écouler les produits hors de la trémie 4 et hors de l'enceinte 8.

Un support cylindrique 2 est placé à une distance d sous la face inférieure 8a de l'enceinte. Ce cylindre est monté rotatif autour d'un axe A-A' orthogonal à l'axe Z-Z' de la trémie. Le support pourrait prendre une autre forme que cylindrique, par exemple il pourrait être sphérique. Selon les applications, la distance d est comprise entre 1 et 3 cm, sans que ceci soit limitatif.

A partir de la face inférieure 8a de l'enceinte et à la périphérie de l'orifice 4a, en direction du cylindre 2, s'étend un ensemble de composants (par exemple des fils, des filaments, des poils, des lamelles, sans que ceci soit limitatif) souples formant une brosse 3. Les composants de la brosse 3 forment une paroi souple qui définit un volume dans lequel les produits 5 présents dans la trémie 4 peuvent tomber par gravité dans une cavité du support 2 : ce volume met donc en communication l'ouverture 4a de la trémie et la cavité. La forme de ce volume peut être quelconque : cylindrique, conique... Cette brosse 3 se distingue donc des brosses connues en ce qu'elle est en quelque sorte « creuse » et forme un canal souple dans lequel les produits peuvent s'écouler de l'orifice 4a de la trémie 4 vers une cavité 1a, 1b sans se disperser.

Tel que représenté, le cylindre 2 comporte deux cavités 1a, 1b à sa surface, la cavité 1b étant masquée par la brosse 3. Les cavités 1a, 1b sont de forme quelconque mais de préférence hémisphérique ; pour un plus grand volume, elles pourraient s'étendre le long d'une génératrice du cylindre 2. Le nombre de cavités à la surface du support 2 pourrait être différent de deux.

La brosse 3 remplit deux fonctions :
- Grâce au volume qu'elle définit, elle guide les produits fragiles 5 de l'orifice 4a vers une cavité 1a, 1b lorsque celle-ci se trouve en face de l'orifice 4a ; elle évite ainsi la dispersion des produits fragiles entre la trémie 4 et le support 2,
- Elle réduit l'effort de cisaillement exercé sur les produits fragiles lorsque le cylindre 2 tourne, réduisant ainsi les risques d'endommagement desdits produits fragiles.

La longueur des composants de la brosse 3 est telle que les extrémités libres de ces composants sont en permanence en contact avec la surface du support 2 et la balayent dans sa rotation. Les composants de la brosse 3 ne sont donc pas forcément tous de la même longueur. Les extrémités libres de ces composants forment une courbe qui reproduit la forme de l'intersection du volume défini par la brosse 3 et du support 2 afin d'une part de garantir une bonne étanchéité entre la brosse 3 et le support 2, d'autre part de limiter les efforts de frottement sur les produits fragiles. Ainsi, dans l'hypothèse où la brosse 3 définit un volume de forme cylindrique à base circulaire :
- Dans le cas où le support 2 est un cylindre, les extrémités libres des composants de la brosse 3 forment une courbe gauche qui est celle de l'intersection de deux cylindres, en conséquence les composants de la brosse 3 n'ont pas tous la même longueur,
- Dans le cas où le support 2 est une sphère, les extrémités libres des composants de la brosse 3 forment une courbe qui est celle de l'intersection d'un cylindre et d'une sphère, c'est-à-dire un cercle (pour autant que l'axe du cylindre passe par le centre de la sphère) et dans ce cas les composants de la brosse 3 ont tous la même longueur.

De plus, la disposition de ces composants est telle que, lorsque l'ouverture 4a est en face d'une cavité 1a, 1b, leur extrémité libre entoure la périphérie de la cavité, de façon à éviter toute dispersion de produits fragiles entre la brosse et le support 2.

Lorsque le support 2 poursuit sa rotation, les produits 5 qui étaient tombés dans une cavité 1a, 1b sont balayés par la brosse 3 et peuvent s'échapper de la cavité. Dans la figure 2, le support 2 a entamé sa rotation et la fraction de produits 5a qui sont tombés dans la cavité la peuvent commencer à s'en échapper (flèche F).

La figure 1 représente des groupes de tels produits 5a, recueillis dans un moyen d'éjection 7 prenant ici la forme d'un entonnoir. L'entonnoir comporte une canalisation de sortie 7a guidant les groupes 5a de produits vers l'extérieur du dispositif 10. Ce moyen d'éjection 7 et sa canalisation de sortie 7a sont avantageux dans le cas où le dispositif est embarqué dans un moyen de transport tel qu'un aéronef pour assurer une transition « douce » aux produits entre l'intérieur du dispositif et l'extérieur. La figure 1 représente également un groupe 5b de tels produits qui sont sortis du dispositif 10.

Le dispositif 10 permet ainsi de larguer des quantités précisément dosées de produits fragiles dans l'environnement et sans les endommager. On comprend qu'il est aisé de jouer sur le dosage en modifiant la forme, le volume et/ou le nombre des cavités 1a, 1b ainsi que sur la vitesse de rotation du support 2. De plus, cette vitesse de rotation n'est pas forcément constante, celui-ci pouvant avancer pas à pas.

Des produits fragiles à larguer sont par exemple des semences, des biocides, des spores bactériens (comme la sporulation en cristal protéique toxique pour les insectes de par exemple *Bacillus thuringiensis israelensis* (Bti) et *Bacillus sphaericus* (Bs)), mais le dispositif est avantageusement utilisé pour larguer de façon précisément calibrée des insectes irradiés pour être stériles ou génétiquement modifiés en vue de la lutte contre certaines maladies telles que le paludisme et arboviroses transmises par les moustiques ou les trypanosomoses africaines transmises par la glossine (mouche tsé-tsé). Les insectes largués entrent en concurrence avec les insectes « naturels » mais n'ont pas de descendance. Il peut également s'agir de parasitoïdes.

Dans ce cas, il est utile que les insectes soient maintenus à une température relativement basse, comprise entre 8 et 10°C, et donc en léthargie, dans la trémie 4. Dans ce cas l'enceinte 8 forme une barrière thermique et le dispositif 10 comporte un moyen pour fournir du froid ou, de façon alternative, il est adapté pour recevoir des corps froids tels que des packs à changement de phase à une température donnée et/ou de la carboglace.

Les insectes n'ont pas tous la même fragilité et les composants de la brosse 3 sont avantageusement adaptés au type d'insecte à larguer. Par exemple, la glossine est une mouche relativement résistante et les poils ou filaments de la brosse 3 peuvent être relativement rigides. En revanche les moustiques sont fragiles et les « poils » de la brosse 3 devront être beaucoup plus souples afin de ne pas les blesser. Les composants de la brosse 3 peuvent être choisis parmi des fibres naturelles (soies de sanglier...) ou artificielles. Il peut aussi s'agir de lamelles souples. L'homme du métier sait choisir le type de fibre ou de lamelles adapté à tel ou tel insecte.

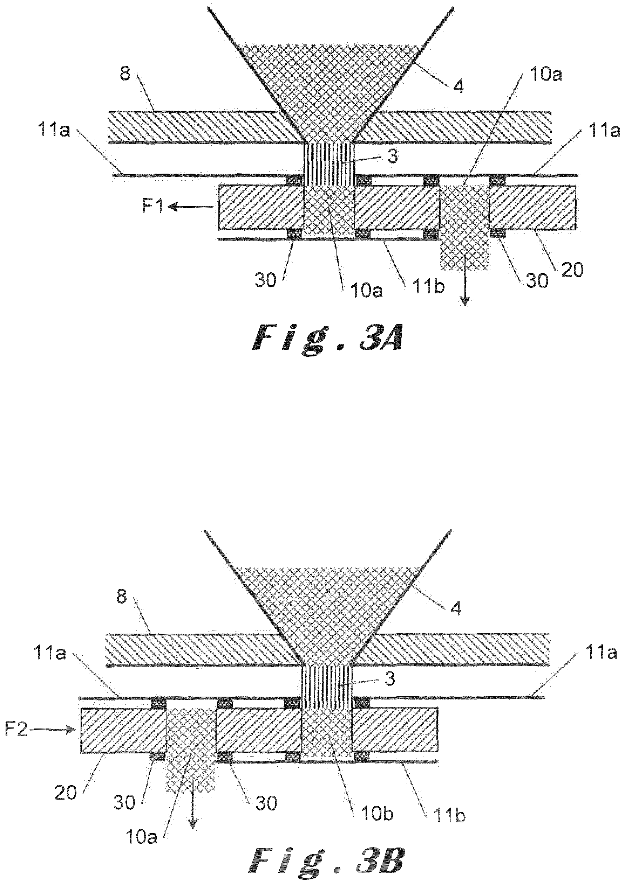

Les figures 3A et 3B illustrent un autre mode de réalisation du dispositif. Toute la partie amont du dispositif est identique à l'exemple des figures 1 et 2 : trémie 4, enceinte 8, brosse 3. En revanche, dans ce mode de réalisation, le support ne se déplace pas par rotation mais par translation et il est composé d'une plaque 20.

Dans l'exemple illustré, la plaque 20 comporte deux cavités traversantes 10a, 10b. La plaque 20 est animée d'un mouvement de va et vient de façon à présenter en face de l'orifice 4a de la trémie 4 tantôt la première cavité 10a, tantôt la seconde cavité 10b. Lorsque la première cavité 10a se trouve en face de l'orifice 4a (figure 3A), elle se remplit en produits fragiles et la seconde cavité 10b se vide. La plaque 20 se déplace ensuite vers la gauche (flèche F1) pour se retrouver dans la position de la figure 3B, dans laquelle la cavité 10a se vide et la cavité 10b se remplit. Puis la plaque 20 revient dans la position de la figure 1 en se déplaçant à droite (flèche F2).

Pour que la cavité qui se trouve en face de l'orifice 4a se remplisse sans se vider en même temps, et pour guider les produits hors de la cavité qui doit se vider, la plaque 20 coulisse entre deux guides fixes, un guide supérieur 11a et un guide inférieur 11b. Le guide supérieur 11a comporte une ouverture traversante placée en face de l'orifice 4a de façon à permettre le remplissage de la cavité placée en face de l'orifice 4a et s'étend largement de part et d'autre de cette ouverture. Au contraire, le guide inférieur 11b comporte une partie pleine placée en face de l'orifice 4a.

De cette façon :
- Comme illustré en figure 3A, la première cavité 10a se remplit de produits fragiles, ces derniers ne pouvant s'échapper car la cavité est fermée dans sa partie inférieure par le guide inférieur 11b, pendant que la seconde cavité 10b se vide, les produits étant empêchés de s'échapper par la partie supérieure de la cavité par l'aile droite du guide 11b,
- La figure 3B illustre la situation miroir dans laquelle la seconde cavité 10b se remplit de produits fragiles, ces derniers ne pouvant s'échapper car la cavité est fermée dans sa partie inférieure par le guide inférieur 11b, pendant que la première cavité 10a se vide, les produits étant empêchés de s'échapper par la partie supérieure de la cavité par faite gauche du guide 11b.

Pour éviter de blesser les produits fragiles lors des mouvements de tiroir du support 20, la périphérie des ouvertures 10a, 10b sont avantageusement munies de brossettes 30.

Dans une variante de ce mode de réalisation, le support 20 pourrait être circulaire et entraîné en rotation autour d'un axe parallèle à l'axe Z-Z' de la trémie.

## Revendications

1. Dispositif (10) pour le largage de produits fragiles (5) comprenant :
- un moyen de stockage (4) desdits produits, comportant un moyen d'évacuation (4a) desdits produits,
- un moyen de distribution des produits fragiles comportant au moins une cavité (1a, 1b, 10a, 10b) ménagée à la surface d'un support (2, 20), le support étant apte à prendre au moins deux positions, une première position dans laquelle l'au moins une cavité se trouve en face de et à distance dudit moyen d'évacuation et peut se remplir en produits fragiles, et une seconde position dans laquelle ladite cavité est écartée dudit moyen d'évacuation et peut se vider,
- -un moyen de mise en communication souple (3) du moyen d'évacuation et de ladite au moins une cavité du support (2) pour le remplissage en produits fragiles de ladite cavité,
**caractérisé en ce que** ledit moyen de mise en communication (3) définit un volume reliant le moyen d'évacuation (4a) et le support (2).

2. Dispositif pour le largage de produits fragiles selon la revendication 1, **caractérisé en ce que** le moyen de mise en communication (3) s'étend à partir de la périphérie du moyen d'évacuation (4a).

3. Dispositif pour le largage de produits fragiles selon l'une des revendications précédentes, **caractérisé en ce que** le moyen de mise en communication (3) comporte des composants souples prévus pour assurer le guidage des produits (5) lorsqu'ils tombent dans une cavité (1a, 1b, 10a, 10b) se trouvant en face du moyen d'évacuation (4a).

4. Dispositif pour le largage de produits fragiles selon la revendication 3, **caractérisé en ce que** l'extrémité libre des composants souples est en contact avec la surface du support (2), notamment avec la périphérie d'une cavité (1a, 1b, 10a, 10b) lorsque cette cavité est en face du moyen d'évacuation (4a).

5. Dispositif pour le largage de produits fragiles selon l'une des revendications 3 ou 4, **caractérisé en ce que** les composants souples sont des fils, des filaments, des poils ou des lamelles.

6. Dispositif pour le largage de produits fragiles selon l'une des revendications précédentes, **caractérisé en ce que** le support (2) est une sphère montée rotative ou un cylindre monté rotatif autour d'un axe (A-A').

7. Dispositif pour le largage de produits fragiles selon l'une des revendications précédentes, **caractérisé en ce que** l'au moins une cavité (1a, 1b) est de forme hémisphérique.

8. Dispositif pour le largage de produits fragiles selon l'une des revendications précédentes, **caractérisé en ce qu'**il comporte un moyen d'éjection (7) apte à recueillir les produits (5) qui sont sortis d'une cavité (1a, 1b, 10a, 10b) et à les guider vers l'extérieur du dispositif.

9. Dispositif pour le largage de produits fragiles selon l'une des revendications précédentes, **caractérisé en ce qu'**il comporte une enceinte (8) formant une forme une barrière thermique, ladite enceinte renfermant le moyen de stockage (4).

10. Dispositif pour le largage de produits fragiles selon l'une des revendications précédentes, **caractérisé en ce qu'**il comporte en outre un moyen de commande prévu notamment pour ajuster la vitesse de déplacement du support (2).

11. Véhicule tel qu'un aéronef comportant un dispositif (10) selon l'une des revendications précédentes.

12. Procédé pour larguer des insectes à l'aide d'un dispositif (10) selon l'une des revendications 1 à 10, **caractérisé en ce qu'**il comporte les étapes consistant à :
- Remplir le moyen de stockage (4) d'insectes,
- Activer le support (2) de façon à larguer des insectes.

13. Procédé pour larguer des insectes selon la revendication 12, **caractérisé en ce qu'**il comporte une étape préalable consistant à refroidir le moyen de stockage (4) de façon à mettre ou maintenir les insectes en léthargie.

14. Procédé pour larguer des insectes selon l'une des revendications 12 ou 13, **caractérisé en ce qu'**il comporte une étape préalable consistant à embarquer le dispositif dans un véhicule tel qu'un aéronef de façon à effectuer le largage d'insectes en vol.

15. Procédé pour larguer des insectes selon l'une des revendications 12 à 14, **caractérisé en ce que** les insectes stérilisés par irradiation ou génétiquement modifiés afin que leur descendance soit non viable.

## Patentansprüche

1. Vorrichtung (10) zum Abwerfen zerbrechlicher Produkte (5), die umfasst:
- ein Lagermittel (4) der Produkte, das ein Evakuierungsmittel (4a) der Produkte umfasst,
- ein Verteilungsmittel der zerbrechlichen Produkte, das mindestens einen Hohlraum (1a, 1b, 10a, 10b) umfasst, der auf der Oberfläche eines Trägers (2, 20) angelegt ist, wobei der Träger geeignet ist, mindestens zwei Positionen einzunehmen, eine erste Position, in der sich der mindestens eine Hohlraum gegenüber und beabstandet von dem Evakuierungsmittel befindet und sich mit zerbrechlichen Produkten füllen kann, und eine zweite Position, in der der Hohlraum abseits von dem Evakuierungsmittel ist und sich entleeren kann,
- ein Mittel zum Herstellen einer biegsamen Verbindung (3) des Evakuierungsmittels und des mindestens einen Hohlraums des Trägers (2) für das Füllen des Hohlraums mit zerbrechlichen Produkten,
**dadurch gekennzeichnet, dass** das Mittel zum Herstellen einer biegsamen Verbindung (3) einen Raum definiert, der das Evakuierungsmittel (4a) und den Träger (2) verbindet.

2. Vorrichtung zum Abwerfen zerbrechlicher Produkte nach Anspruch 1, **dadurch gekennzeichnet, dass** sich das Mittel zum Herstellen einer Verbindung (3) ausgehend von der Peripherie des Evakuierungsmittels (4a) erstreckt.

3. Vorrichtung zum Abwerfen zerbrechlicher Produkte nach einem der vorstehenden Ansprüche, **dadurch gekennzeichnet, dass** das Mittel zum Herstellen einer Verbindung (3) biegsame Bauteile umfasst, die vorgesehen sind, um die Führung der Produkte (5) sicherzustellen, wenn sie in einen Hohlraum (1a, 1b, 10a, 10b) fallen, der gegenüber dem Evakuierungsmittel (4a) liegt.

4. Vorrichtung zum Abwerfen zerbrechlicher Produkte nach Anspruch 3, **dadurch gekennzeichnet, dass** das freie Ende der biegsamen Bauteile mit der Oberfläche des Trägers (2), insbesondere mit der Peripherie eines Hohlraums (1a, 1b, 10a, 10b) in Kontakt ist, wenn dieser Hohlraum gegenüber dem Evakuierungsmittel (4a) liegt.

5. Vorrichtung zum Abwerfen zerbrechlicher Produkte nach einem der Ansprüche 3 oder 4, **dadurch gekennzeichnet, dass** die biegsamen Bauteile Drähte, Filamente, Flor oder Lamellen sind.

6. Vorrichtung zum Abwerfen zerbrechlicher Produkte nach einem der vorstehenden Ansprüche, **dadurch gekennzeichnet, dass** der Träger (2) eine Kugel ist, die drehend montiert ist, oder ein Zylinder, der um eine Achse (A-A') drehend montiert ist.

7. Vorrichtung zum Abwerfen zerbrechlicher Produkte nach einem der vorstehenden Ansprüche, **dadurch gekennzeichnet, dass** der mindestens eine Hohlraum (1a, 1b) eine Halbkugelform aufweist.

8. Vorrichtung zum Abwerfen zerbrechlicher Produkte nach einem der vorstehenden Ansprüche, **dadurch gekennzeichnet, dass** sie ein Auswurfmittel (7) umfasst, das zum Auffangen der Produkte (5), die aus einem Hohlraum (1a, 1b, 10a, 10b) ausgetreten sind, und zu ihrem Führen aus der Vorrichtung heraus geeignet ist.

9. Vorrichtung zum Abwerfen zerbrechlicher Produkte nach einem der vorstehenden Ansprüche, **dadurch gekennzeichnet, dass** sie einen Einschluss (8) umfasst, der eine Wärmebarriere bildet, wobei der Einschluss das Lagermittel (4) umschließt.

10. Vorrichtung zum Abwerfen zerbrechlicher Produkte nach einem der vorstehenden Ansprüche, **dadurch gekennzeichnet, dass** sie weiter ein Steuermittel umfasst, das insbesondere vorgesehen ist, um die Bewegungsgeschwindigkeit des Trägers (2) einzustellen.

11. Fahrzeug, wie ein Luftfahrzeug, das eine Vorrichtung (10) nach einem der vorstehenden Ansprüche umfasst.

12. Verfahren zum Abwerfen von Insekten mit Hilfe einer Vorrichtung (10) nach einem der Ansprüche 1 bis 10, **dadurch gekennzeichnet, dass** es die Schritte umfasst, die bestehen aus:
- Füllen des Lagermittels (4) mit Insekten,
- Aktivieren des Trägers (2) derart, dass die Insekten abgeworfen werden.

13. Verfahren zum Abwerfen von Insekten nach Anspruch 12, **dadurch gekennzeichnet, dass** es einen Vorabschritt umfasst, der darin besteht, das Lagermittel (4) derart abzukühlen, dass die Insekten in Lethargie versetzt oder gehalten werden.

14. Verfahren zum Abwerfen von Insekten nach einem der Ansprüche 12 oder 13, **dadurch gekennzeichnet, dass** es einen Vorabschritt umfasst, der darin besteht, die Vorrichtung in einem Fahrzeug, wie in einem Luftfahrzeug, derart mitzuführen, dass das Abwerfen von Insekten während des Flugs ausgeführt wird.

15. Verfahren zum Abwerfen von Insekten nach einem der Ansprüche 12 bis 14, **dadurch gekennzeichnet, dass** die Insekten durch Bestrahlung sterilisiert oder genetisch modifiziert sind, damit ihre Nachkommen nicht lebensfähig sind.

## Claims

1. Device (10) for dropping fragile products (5) comprising:
- a storage means (4) of said products, comprising a discharge means (4a) of said products,
- a distribution means of the fragile products comprising at least one cavity (1a, 1b, 10a, 10b) provided at the surface of a support (2, 20), the support being capable of taking at least two positions, a first position wherein the at least one cavity is located facing and at a distance of said discharge means and can be filled with fragile products, and a second position wherein said cavity is spaced apart from said discharge means and can be emptied,
- a flexible means of communication (3) of the discharge means with said at least one cavity of the support (2) in order to fill said cavity with fragile products,
**characterised in that** said communication means (3) defines a volume connecting the discharge means (4a) and the support (2).

2. Device for dropping fragile products according to claim 1, **characterised in that** the communication means (3) extends from the periphery of the discharge means (4a).

3. Device for dropping fragile products according to one of the preceding claims, **characterised in that** the communication means (3) comprises flexible components provided to ensure the guiding of the products (5) when they fall in a cavity (1a, 1b, 10a, 10b) located facing the discharge means (4a).

4. Device for dropping fragile products according to claim 3, **characterised in that** the free end of the flexible components is in contact with the surface of the support (2), particularly with the periphery of a cavity (1a, 1b, 10a, 10b) when this cavity is facing the discharge means (4a).

5. Device for dropping fragile products according to one of claims 3 or 4, **characterised in that** the flexible components are threads, filaments, hairs or thin strips.

6. Device for dropping fragile products according to one of the preceding claims, **characterised in that** the support (2) is a sphere rotationally mounted, or a cylinder rotationally mounted about an axis (A-A').

7. Device for dropping fragile products according to one of the preceding claims, **characterised in that** the at least one cavity (1a, 1b) is of a hemispherical shape.

8. Device for dropping fragile products according to one of the preceding claims, **characterised in that** it comprises an ejection means (7) capable of collect the products (5) that have exited a cavity (1a, 1b, 10a, 10b) and to guide them towards the exterior of the device.

9. Device for dropping fragile products according to one of the preceding claims, **characterised in that** it comprises an enclosure (8) that forms a thermal barrier, said enclosure enclosing the storage means (4).

10. Device for dropping fragile products according to one of the preceding claims, **characterised in that** it further comprises a control means provided particularly to adjust the speed of motion of the support (2).

11. Vehicle such as an aircraft comprising a device (10) according to one of the preceding claims.

12. Method for dropping insects using a device (10) according to one of the claims 1 to 10, **characterised in that** it comprises the steps consisting of:
- Filling the storage means (4) with insects,
- Activating the support (2) so as to drop the insects.

13. Method for dropping insects according to claim 12, **characterised in that** it comprises a prior step consisting of cooling the storage means (4) so as to make or keep the insects lethargic.

14. Method for dropping insects according to one of claims 12 or 13, **characterised in that** it comprises a prior step consisting of loading the device into a vehicle such as an aircraft so as to perform the insect drop in flight.

15. Method for dropping insects according to one of the claims 12 to 14, **characterised in that** the insects are sterilised by irradiation or genetically modified so that the offspring thereof is not viable.
